(19) Europäisches Patentamt
European Patent Office
Office européen des brevets

(11) Veröffentlichungsnummer: **0 182 271**
**B1**

(12) **EUROPÄISCHE PATENTSCHRIFT**

(45) Veröffentlichungstag der Patentschrifft: **17.01.90**

(21) Anmeldenummer: **85114405.5**

(22) Anmeldetag: **13.11.85**

(51) Int. Cl.⁴: **C 07 D 471/22**, A 61 K 31/475 // (C07D471/22, 221:00, 221:00, 221:00, 209:00)

(54) **Neue Nb-quartäre Dibromderivate von Ajmalin, Isoajmalin, Sandwicin und Isosandwicin sowie diese Derivate enthaltende pharmazeutische Zubereitungen und Zwischenprodukte und Verfahren zu ihrer Herstellung.**

(30) Priorität: **22.11.84 DE 3442452**

(43) Veröffentlichungstag der Anmeldung: **28.05.86 Patentblatt 86/22**

(45) Bekanntmachung des Hinweises auf die Patenterteilung: **17.01.90 Patentblatt 90/03**

(84) Bennante Vertragsstaaten: **BE CH DE FR GB IT LI NL**

(56) Entgegenhaltungen:
**EP-A-0 029 115**
**EP-A-0 029 116**

(73) Patentinhaber: **Kali-Chemie Pharma GmbH**
**Hans-Böckler-Allee 20 Postfach 220**
**D-3000 Hannover 1 (DE)**

(72) Erfinder: **Kehrbach, Wolfgang, Dr.rer.nat., Dipl.-Chem.**
**Altenbekener Damm 41**
**D-3000 Hannover 1 (DE)**
Erfinder: **Schön, Uwe, Dr. Dipl.-Chem.**
**Föhrenkamp 12**
**D-3167 Burgdorf (DE)**

(74) Vertreter: **Lauer, Dieter, Dr.**
**c/o Kali-Chemie AG Postfach 220 Hans-Böckler-Allee 20**
**D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 182 271 B1

LIBERGRAF, STOCKHOLM 1990

**Beschreibung**

Aus der europäischen Patentanmeldung Veröffentlichungs-Nr. 0 029 115 sind $N_b$-quartäre Derivate von 10-Bromajmalin und 10-Bromisoajmalin mit herzrhythmisierenden Eigenschaften bekannt und aus der europäischen Patentanmeldung Veröffentlichungs-Nr. 0 029 116 sind $N_b$-quartäre Derivate von 10-Bromsandwicin und 10-Bromisosandwicin mit herzrhythmisierenden Wirkungen bekannt.

Der vorliegenden Erfindung liegt die Aufgabe zugrunde, neue $N_b$-quartäre bromierte Derivate des Ajmalin. Isoajmalin, Sandwicin und Isosandwicin mit wertvollen pharmakologischen Eigenschaften zu entwickeln.

Es wurde nun gefunden, daß die neuen $N_b$-quartären dibromierten Derivate des Ajmalin, Isoajmalin, Sandwicin und Isosandwicin wertvolle pharmakologische Eigenschaften besitzen und herzrhythmisierende, insbesondere antiarrhythmische Wirkungen sowie daneben auch blutplättchenaggregationshemmende und antithrombotische Wirkungen zeigen und sich durch ein vorteilhaftes Wirkungsprofil mit guter therapeutischer Breite, langer Wirkungsdauer und geringer Toxizität auszeichnen. Aufgrund dieser Wirkungen sind die neuen Verbindungen als Arzneimittel, insbesondere zur Behandlung von Herzrhythmusstörungen sowie zur Prophylaxe und Behandlung von Blutplättchenaggregationen und Throm bosen geeignet.

Die vorliegende Erfindung betrifft daher neue $N_b$-quartäre Verbindungen der allgemeinen Formel I

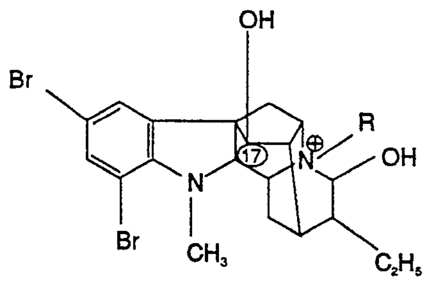

$A^{\ominus}$

I

worin

R für eine Alkylgruppe mit 1 bis 6 Kohlenstoffatomen steht, welche gegebenenfalls an einem nicht an Stickstoff gebundenen Kohlenstoffatom substituiert sein kann durch Hydroxy oder Halogen,
für eine Alkenylgruppe mit 3 bis 6 Kohlenstoffatomen, deren Doppelbindung nicht direkt an das Stickstoffatom gebunden ist,
für eine Phenylalkylgruppe mit 2 bis 3 Kohlenstoffatomen in der Alkylenkette, welche gegebenenfalls im Phenylring durch Alkoxy mit 1 - 3 Kohlenstoffatomen oder Halogen substituiert sein kann, oder für eine Gruppe a steht,

```
   R1
  /
Z-N
  \
   R2
```

a

worin

Z eine Alkylengruppe mit 2 - 3 Kohlenstoffatomen oder die 2-Hydroxypropylenkette bedeutet,

$R_1$ und $R_2$ je Alkyl mit 1 - 3 Kohlenstoffatomen bedeuten oder

$R_2$ und $R_2$ zusammen mit dem Stickstoffatom, an das sie gebunden sind, einen Heterocyclus aus der Gruppe Pyrrolidin, Piperidin, Azepin und Morpholin darstellen, oder, falls Z eine Alkylenkette ist, $R_1$ Alkyl mit 1 - 3 Kohlenstoffatomen und $R_2$ zusammen mit dem Stickstoffatom, an welches es gebunden ist, und dem diesem Stickstoffatom benachbarten Kohlenstoffatom der Kette Z einen Heterocyclus aus der Gruppe Pyrrolidin und Piperidin darstellt, und

$A^-$ das Anion einer physiologisch verträglichen anorganischen oder organischen Säure bedeutet.

Falls in den $N_b$-quartären Verbindungen der Formel I R eine Alkylgruppe bedeutet, kann diese, geradkettig oder verzweigt sein und kann 1 - 6, vorzugsweise 2 - 5, Kohlenstoffatome enthalten und ist vorzugsweise unsubstituiert. Eine Alkenylgruppe R stellt vorzugsweise die Allylgruppe dar. Falls R eine Phenylalkylgruppe darstellt, enthält deren Alkylenkette 2 oder 3 Kohlenstoffatome. Der Phenylring ist vorzugsweise unsubstituiert. Falls der Phenylring durch niederes Alkoxy substituiert ist, kann dieses 1 - 3 Kohlenstoffatome enthalten und stellt bevorzugt Methoxy dar.

In der Gruppe a enthaltene niedere Alkylreste $R_1$ und $R_2$ besitzen 1 - 3, insbesondere 2 Kohlenstoffatome. Als Beispiele von aus dem Rest $R_2$ und dem Stickstoffatom, an welches er gebunden ist, zusammen mit dem Rest $R_1$ oder dem dem Stickstoff benachbarten C der Alkylenkette Z gebildeten heterocyclischen Ringen seien genannt Pyrrolidin, Piperidin, Azepin, Morpholin oder N-Methyl-2-pyrrolidin.

$A^-$ ist das Anion einer physiologisch verträglichen anorganischen oder organischen Säure. Als Anionen anorganischer Säuren eignen sich z. B. Halogenide, insbesondere Chlorid, Sulfate oder Phosphate. Als organische Säuren eignen sich z. B. niedere aliphatische ein- oder mehrwertige Carbonsäuren, wie beispielsweise Weinsäure, Oxalsäure, Zitronensäure, Fumarsäure, Essigsäure oder Propionsäure.

Als pharmakologische Wirkstoffe können die $N_b$-quartären Verbindungen der Formel I oder deren Gemische verwendet werden. Zum Beispiel eignen sich Gemische aus Dibromajmalinderivaten der Formel I, worin $C_{17}$ R-konfiguriert und $C_{20}$ S-konfiguriert vorliegen, mit entsprechenden Dibromisoajmalinderivaten, worin $C_{17}$ R-konfiguriert und $C_{20}$ R-konfiguriert vorliegen, oder Gemische aus Dibromsandwicinderivaten, worin $C_{17}$ S-konfiguriert und $C_{20}$ S-konfiguriert vorliegen, mit den entsprechenden Dibromisosandwicinderivaten, worin $C_{17}$ S-konfiguriert und $C_{20}$ R-konfiguriert vorliegen.

Als zweckmäßig erweisen sich z. B. $N_b$-quartäre Dibromajmalin- und/oder Dibromisoajmalinderivate, worin R für eine Alkylgruppe mit vorzugsweise 2 - 5, insbesondere 2 oder 3, Kohlenstoffatomen oder eine Gruppe a steht, worin Z die oben angegebene Bedeutung besitzt und $R_1$ und $R_2$ je Alkyl mit 1 - 3, vorzugsweise 1 oder 2 Kohlenstoffatomen, bedeuten oder zusammen mit dem Stickstoff, an welchen sie gebunden sind, einen der vorgenannten 5- bis 6-gliedrigen Heterocyclen darstellen oder $R_1$ niederes Alkyl, vorzugsweise Methyl, bedeutet und $R_2$ zusammen mit dem Stickstoff und dem dazu benachbarten C der Alkylenkette Z einen der vorgenannten Heterocyclen darstellt.

Unter den $N_b$-quartären Dibromsandwicin- und/oder Dibromisosandwicinderivaten eignen sich insbesondere solche, worin R Alkyl mit 2 - 5, vorzugsweise 2 oder 3, C-Atomen bedeutet oder für die Gruppe a steht, vorzugsweise für eine Gruppe a, worin Z die oben angegebene Bedeutung besitzt, und $R_1$ und $R_2$ je Alkyl mit 1 - 3 vorzugsweise 1 oder 2 Kohlenstoffatomen bedeuten oder $R_2$ und das Stickstoffatom, an welches es gebunden ist, zusammen mit $R_1$ oder mit dem dem N benachbarten C der Alkylenkette Z einen Heterocyclus bildet.

Die neuen $N_b$-quartären Verbindungen der Formel I können nach an sich bekannten Methoden erhalten werden, indem man a) Verbindungen der allgemeinen Formel II

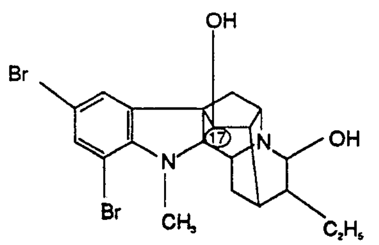

II

mit Verbindungen der allgemeinen Formel III

$$R - X \qquad III$$

worin R obige Bedeutung besitzt und X einen aminolytisch abspaltbaren Rest bedeutet, umsetzt, oder

b) zur Herstellung solcher Verbindungen der Formel I, worin R eine Hydroxygruppe in ß-Stellung zu dem Stickstoffatom des Grundgerüstes enthält, - das sind Verbindungen der Formel I, worin R eine $-CH_2-CHOH-R'$-Gruppe, worin R' Wasserstoff, Alkyl mit 1 - 4 Kohlenstoffatomen oder einen Rest $-CH_2-NR_1R_2$, worin $R_1$ und $R_2$ obige Bedeutung besitzen, bedeutet, darstellt -, Verbindungen der Formel II mit einem Epoxid der allgemeinen Formel IV

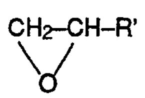

IV

worin R' obige Bedeutung besitzt, zu entsprechenden ringoffenen Aldehydbasen der allgemeinen Formel Va

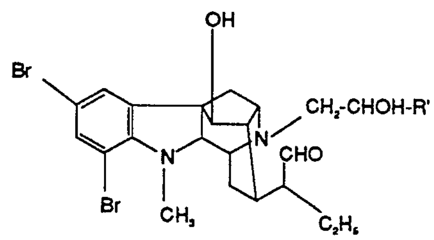

Va

worin R' obige Bedeutung besitzt, umsetzt und diese durch Behandlung mit einer Säure HA, worin A obige Bedeutung besitzt, in die entsprechenden Verbindungen der Formel I überführt.

Die Umsetzung der Verbindungen der Formel II mit Verbindungen der Formel III gemäß Verfahrensvariante a) kann nach an sich zur Alkylierung von Aminen üblichen Methoden erfolgen. Als aminolytisch abspaltbare Reste in den Verbindungen der Formel III kommen insbesondere Halogene wie Chlor, Brom oder Jod, in Frage sowie auch organische Sulfonsäurereste, insbesondere Reste von Niederalkansulfonsäuren wie z. B. Methansulfonsäure oder Äthansulfonsäure oder von aromatischen Sulfonsäuren, insbesondere Benzolsulfonsäuren, welche gegebenenfalls im Benzolring durch niederes Alkyl oder Halogen substituiert sein können, z. B. Benzolsulfonsäure, Toluolsulfonsäuren oder Brombenzolsulfonsäuren. In Verbindungen der Formel III, welche einen basischen stickstoffhaltigen Rest enthalten, kommt als aminolytisch abspaltbare Gruppe bevorzugt Chlor, sonst bevorzugt Brom, infrage. Die Ausgangsstoffe können in äquimolaren Mengen eingesetzt werden, es kann jedoch vorteilhaft sein, die Verbindung der Formel III im Überschuß anzuwenden. Die Umsetzung erfolgt zweckmäßigerweise in einem gegenüber den Reaktionsteilnehmern inerten organischen Lösungsmittel. Als Beispiele geeigneter Lösungsmittel seien genannt aromatische Kohlenwasserstoffe wie Benzol oder Toluol, cyclische Äther wie Dioxan, halogenierte Kohlenwasserstoffe wie Chloroform, niedere Alkohole wie z. B. Methanol oder Äthanol, Acetonitril, Dimethylformamid, Tetramethylharnstoff oder Sulfolan oder deren Gemische. Im Falle von nicht stickstoffhaltigen Verbindungen der Formel III erweisen sich stark polare aprotische Lösungsmittel als besonders vorteilhaft, während bei der Umsetzung mit stickstoffhaltigen Verbindungen der Formel III niedere Alkohole, insbesondere Äthanol bevorzugt sind. Gegebenenfalls kann auch ein Überschuß der Verbindung der Formel III als Lösungsmittel dienen. Die Umsetzung wird beispielsweise bei Temperaturen zwischen 0 und 150°C, vorzugsweise 20 - 100°C durchgeführt, wobei der jeweils günstigste Temperaturbereich je nach Art der eingesetzten Reaktionsteilnehmer und des verwendeten Lösungsmittels verschieden sein kann, z. B. können die Temperaturen umso niedriger gewählt werden, je stärker polar das verwendete Lösungsmittel ist.

Die Umsetzungen der Verbindungen der Formel II mit Epoxidverbindungen der Formel IV kann nach an sich zur Anlagerung von Epoxiden an Amine üblichen Methoden erfolgen. Die Ausgangsstoffe können dabei in äquimolaren Mengen eingesetzt werden oder es kann ein Überschuß des Epoxids der Formel IV vorhanden sein. Zweckmäßig wird die Umsetzung in einem gegenüber den Reaktionsteilnehmern inerten protischen organischen Lösungsmittel, beispielsweise einem niederen Alkohol durchgeführt. Die Umsetzung findet zweckmäßigerweise bei erhöhter Temperatur, beispielsweise bei Temperaturen zwischen 50 und 100°C, vorzugsweise bei Siedetemperatur des Lösungsmittels statt.

Die erhaltenen ringoffenen Aldehydbasen der Formel Va können ohne vorherige Reinigung durch Behandlung mit Säure in die $N_b$-quartären Verbindungen der Formel I überführt werden.

Bei der Quarternisierung der Ausgangsverbindungen der Formel II kann eine Isomerisierung am Zentrum $C_{20}$ auftreten. Diese Isomerisierung kann sowohl bei Verwendung von sterisch reinen Dibromajmalin- bzw. Dibromsandwicinderivaten oder sterisch reinen Dibromisoajmalin-bzw. Dibromisosandwicinderivaten der Formel II als auch bei Verwendung von Isomerengemischen der Verbindungen der Formel II auftreten und zu einer Verschiebung des Isomerenverhältnisses in dem entsprechenden $N_b$ -quartären Endprodukt der Formel I oder der ringoffenen Aldehydbase gegenüber dem Ausgangsprodukt führen. Das Isomerenverhältnis in dem Reaktionsprodukt kann von der Art des eingeführten Restes R bestimmt werden, kann darüber hinaus jedoch auch durch die Reaktionsbedingungen beeinflußt werden und kann unterschiedlich ausfallen je nach der Reaktionstemperatur, dem verwendeten Lösungsmittel oder je nachdem, ob man reine n-, reine Isoverbindungen oder Isomerengemische als Ausgangsverbindungen der Formel II einsetzt. Auch bei Überführung der ringoffenen Aldehydbasen in entsprechende $N_b$-quartäre Verbindungen der Formel I kann sich das Isomerenverhältnis weiter verändern.

Die aus den vorerwähnten Umsetzungen resultierenden $N_b$-quartären Verbindungen der Formel I bzw. deren Isomeren-Gemische können auf an sich bekannte Weise, aus dem Reaktionsprodukt isoliert und gereinigt, werden, beispielsweise durch chromatographische Reinigung und/oder fraktionierte Kristallisation bzw. Umkristallisa-

tion. Gewünschtenfalls kann das Anion A gegen ein anderes Anion ausgetauscht werden. Hierzu werden die erhaltenen $N_b$-quartären Verbindungen der Formel I durch Behandlung mit Alkalien in die entsprechenden ringoffenen Aldehydbasen der Formel V

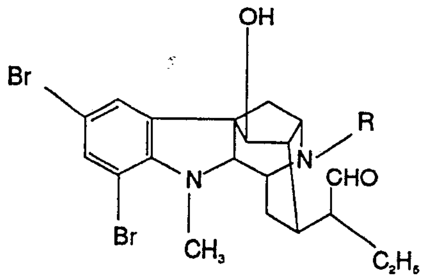

(V)

worin R obige Bedeutung besitzt, überführt und diese durch Umsetzung mit einer Säure HA, worin A die gewünschte Bedeutung besitzt, wieder in eine $N_b$-quartäre Verbindung der Formel I überführt.

Die Umwandlung der $N_b$-quartären Verbindungen der Formel I in die entsprechenden ringoffenen Aldehydbasen der Formel V kann auf an sich, bekannte Weise erfolgen, indem die Verbindungen der Formel I mit wäßrigen Lösungen anorganischer Basen, beispielsweise wäßriger, vorzugsweise etwa 10 %-iger Natronlauge oder wäßriger Natriumhydrogencarbonat oder Natriumcarbonat-Lösungen behandelt werden. Zweckmäßigerweise wird in Gegenwart eines inerten mit Wasser nicht mischbaren organischen Lösungsmittels, in welchem die Aldehydbase ausreichend löslich ist, gearbeitet, wie z. B. halogenierten Kohlenwasserstoffen, wie Dichlormethan, Essigsäureäthylester oder Diäthyläther. Beispielsweise kann das quartäre Salz in Wasser gelöst bzw. eingeschlemmt werden, die Mischung unter Eiskühlung alkalisch gemacht und mit einem geeigneten Lösungsmittel extrahiert werden. Die Wahl des Lösungsmittels wird durch die Löslichkeit der Aldehydbase bestimmt. Bei Aminoalkylderivaten ist es vorteilhaft, ein möglichst unpolares Lösungsmittel zu verwenden, um die Extraktion polarer Nebenprodukte zu vermeiden. Bei schwerlöslichen Aldehydbasen, insbesondere Sandwicin oder Isosandwicinaldehydbasen, kann es andererseits zweckmäßig sein, den Lösungsmitteln, z. B. halogenierten Kohlenwasserstoffen zur Verbesserung des Lösevermögens einen die Polarität erhöhenden Zusatz an niederen Alkoholen, z. B. Methanol, beizugeben. Nach Beendigung der Reaktion wird die organische Phase abgetrennt und die darin enthaltenen rohen Aldehydbasen können ohne weitere Reinigung mit der Säure HA zu $N_b$-quartären Verbindungen der Formel I umgesetzt werden.

Gewünschtenfalls kann zunächst durch Eindampfen des Lösungsmittels die rohe Aldehydbase isoliert und dann durch Behandlung mit einer Säure HA in die gewünschte Verbindung der Formel I überführt werden. Beispielsweise kann die organische Phase eingedampft werden, die zurückbleibende Aldehydbase in einem inerten Lösungsmittel gelöst und langsam unter Rühren mit einer Lösung der berechneten Säuremenge in einem inerten Solvens versetzt werden. Geeignete Lösungsmittel sind z. B. Diäthyläther, Essigester, Aceton, Methylenchlorid und niedere Alkohole wie Methanol, Äthanol oder Isopropanol. In einigen Fällen kann die organische Phase direkt zur Salzbildung verwendet werden. Alternativ kann auch eine Lösung der Säure HA vorgelegt und mit einer Lösung der Aldehydbase versetzt werden. Falls die Salzbildung in polaren Solvenzien, wie z. B Methanol vorgenommen wird und das Salz nicht direkt ausfällt, so kann die Lösung zur Ausfällung des Salzes langsam unter Rühren in ein unpolares Lösungsmittel, wie z. B. Essigsäureäthylester oder Diäthyläther, eingetropft werden.

Die neuen $N_b$-quartären Dibromverbindungen der Formel I zeichnen sich durch interessante pharmakologische Eigenschaften aus. Sie zeigen insbesondere herzrhythmisierende wie auch antithrombotische Wirkungen. Dabei zeichnen sich die neuen durch Verbindungen durch eine gute orale Wirksamkeit, lange Wirkungsdauer und Breite aus.

Die antiarrhythmischen und blutplättchenaggregationshemmenden Wirkungen der Verbindungen lassen sich in pharmakologischen Standardtestmethoden zeigen.

Beschreibung der pharmakologischen Versuchsmethoden:

1. Bestimmung der akuten 7-Tage-Toxizität in Mäusen. Die akute Toxizität der Wirkstoffe wird in männlichen NMRI-Mäusen (Körpergewicht 18 - 22 g) nach Einmaliger p.o.-Applikation bestimmt. Die $LD_{50}$ wird als die Dosis in µMol/kg definiert, welcher eine 50 % Mortalität der Tiere am 7. Tag nach der Applikation entspricht. Die $LD_{50}$-Werte werden durch eine Probit-Analyse errechnet.

2. Bestimmung der Hemmwirkung der Substanzen auf durch Aconitin-Infusion induzierte Herzrhythmusstörungen in Ratten.

Die Hemmwirkung auf durch Aconitin-Infusion in Ratten verursachte Herzrhythmusstörungen (Extrasystolen (ES), ventrikuläre Tachykardie (VT) und Kammerflattern (KF)) werden nach der Methode von Raschak (Arzneimittelforsch. *25* (1975), 639 - 641) bestimmt.

Männliche Ratten mit einem Körpergewicht von 320 - 400 g werden durch i.p. Applikation von 1,25 g/kg Urethan narkotisiert. Die Tiere werden auf den Rücken gelegt und ihr Elektrokardiogramm wird während des Versuches alle 30 Sekunden gemessen. Den Tieren wird eine i.v. Aconitin-Infusion mit einer Infusionsgeschwindigkeit von 5 µg/kg/min Aconitin und einem Infusionsvolumen von 0,1 ml/min verabreicht. Drei Stunden vor Beginn der Aconitin-Infusion werden den Tieren die Testsubstanzen p.o. in einer Menge von 2 ml/kg Körpergewicht 2 %-iger Tylose-Lösung verabreicht. Einer Kontrollgruppe wird nur die Tylose-Lösung verabreicht. Die Infusionsdauer bis zum Auftreten der verschiedenen Herzrhythmusstörungen wird bestimmt.

Antiarrhythmisch wirksame Testverbindungen führen zu einer Verzögerung des Auftretens der Aconitin-induzierten Herzrhythmusstörungen in den behandelten Tieren im Vergleich zu den Kontrolltieren. Aus dem Ausmaß der Verlängerung der Aconitininfusionsdauer und somit der Erhöhung der verabreichten Aconitinmenge, bis zu welcher die Tiere gegen die durch Aconitin induzierten Herzrhythmusstörungen geschützt sind, läßt sich auf die Stärke der antiarrhythmischen Wirksamkeit der Testsubstanzen schließen. Als $ED_{150}$ wird die Dosis der Testsubstanzen bestimmt, bei welcher die jeweiligen Herzrhythmusstörungen erst nach der Verlängerung der Aconitinfusionsdauer auf 150 % auftreten,

3. Bestimmung der Hemmwirkung auf durch ADP induzierte Blutplättchenaggregation im blutplättchenreichen Kaninchenblutplasma (PRP/K).

Die in-vitro-Bestimmung der antiaggregatorischen Wirkungen der Substanzen wurde nach der Methode von Born (Nature *194*, 927 (1962)), modifiziert für Mikrobestimmungen nach Sim et al (Thromb. Res. *7*, 655 (1975)) durchgeführt.

PRP/K wird durch Zentrifugieren von mit Antikoagulanz versetztem Kaninchenblut auf bekannte Weise erhalten. Als Kontrollwerte für die photometrische Bestimmung der Aggregationshemmung werden die optische Dichte des unbehandelten PRP/K (≙ 0 % Aggregation) und von PRP/K, in welchem Blutplättchenaggregation durch Behandlung von $10^{-6}$ Mol/l ADP erzeugt wurde (≙ 100 % Aggregation), gemessen.

Zur Bestimmung der aggregationshemmenden Wirkungen der Wirkstoffe werden Proben PRP/K mit einer bestimmten Wirkstoffdosis versetzt und dann mit $10^{-6}$ Mol/l ADP behandelt. Die Verringerung der optischen Dichte gegenüber der des unbehandelten PRP/K wird gemessen und mit der bei der Behandlung von wirkstoffreiem PRP/K mit $10^{-6}$ Mol/l ADP aufgetretenen optischen Dichteverringerung verglichen. Hieraus wird die durch die eingesetzte Wirkstoffdosis entfaltete Hemmwirkung in % Hemmung der in wirkstoffreiem PRP/K verursachten Aggregation berechnet. Die in diesem Test mit Wirkstoffen der Formel I erhaltenen Ergebnisse werden in der nachstehenden Tabelle II wiedergegeben.

Die folgenden Tabellen I und II geben nach den vorstehend beschriebenen Testmethoden erhaltene Ergebnisse wieder. Die für die Verbindungen der Formel I angegebenen Beispielsnummern beziehen sich auf die nachstehenden Herstellungsbeispiele.

**Tabelle I**

| Bsp. Nr. | Toxizität $LD_{50}$ µmol/kg p.o. Maus | Hemmwirkung gegenüber aconotininduzierten Herzrhythmusstörungen $ED_{150}$ µmol/kg p.o. Ratte | | |
|---|---|---|---|---|
| | | ES | VT | KF |
| 12 | 2400 | 49 | 64 | 58 |
| 22 | > 2150 | 22 | 28 | 30 |
| 33 | > 2150 | 26 | 21 | 28 |
| 2 | 277 | 9 | 6 | 11 |
| 62 | 379 | 11 | 11 | 13 |
| 67 | 720 | 23 | 20 | 23 |
| 38 | > 464 < 1000 | 8 | 8 | 8 |

**Tabelle II**

| Bsp. No. | Hemmwirkung gegenüber ADP-induzierter Plättchenaggregation in PRP-K Dosis µg/ml | % Hemmwirkung |
|---|---|---|
| 16 | 50 | 81 |
| 12 | 100 | 83 |
| 55 | 25 | 57 |
| 62 | 100 | 100 |
| 67 | 5 | 53 |
| 72 | 50 | 77 |
| 69 | 5 | 65 |
| 8 | 100 | 100 |
| 68 | 10 | 54 |

Aufgrund ihrer vorstehend beschriebenen herzwirksamen Eigenschaften eignen sich die $N_b$-quartären Verbindungen der Formel I als Arzneimittel für Menschen und größere Säugetiere zur Behandlung von Herzrhythmusstörungen. Außerdem eignen sich die Verbindungen aufgrund ihrer blutplättchenaggregationshemmenden und Antithrombotischen Wirkung auch als Arzneimittel zur Prophylaxe und Behandlung von Thrombosen. Aufgrund dieser Kombination von herzrhythmisierender Wirksamkeit einerseits und antithrombotischer Wirksamkeit andererseits eignen sich die Verbindungen der Formel I insbesondere zur prophylaktischen und therapeutischen Behandlung von Patienten mit Herzrhythmusbeschwerden, welche gleichzeitig auch an Thrombosen leiden oder thrombosegefährdet sind, beispielsweise zur Behandlung von herzinfarktgefährdeten Patienten oder zur Nachbehandlung von Herzinfarktpatienten.

Die zu verwendenden Dosen können individuell verschieden sein und variieren naturgemäß je nach Art des zu behandelnden Zustandes, des Behandlungsstadiums (z. B. Initialdosis oder Dosis zur Langzeitbehandlung), der verwendeten Substanz und der Applikationsform. z. B. werden parenterale Formulierungen im allgemeinen weniger Wirkstoff enthalten als orale Präparate. Im allgemeinen eignen sich jedoch zur Applikation an Menschen und größeren Säugetieren Tagesdosen von 0,1 - 10, insbesondere 0,5 - 5 mg/kg.

Als Heilmittel können die $N_b$-quartären Verbindungen der Formel I bzw. deren Genische erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Kapseln, Pulver, Granulate oder Dragees genannt, oder auch Suppositorien. Feste Präparate können pharmazeutisch übliche anorganische und/oder organische Trägerstoffe, wie z. B. Talkum, Milchzucker oder Stärke neben weiteren pharmazeutisch üblichen Hilfsmitteln, wie beispielsweise Gleitmitteln wie Magnesiumstearat oder Tablettensprengmitteln, enthalten. Flüssige Präparate wie Lösungen, Suspensionen oder Emulsionen können die üblichen Verdünnungsmittel wie Wasser, Öle oder Paraffine und/oder Suspensionsmittel, wie Polyoxyäthylenglycol und dergl. enthalten. Es können zusätzlich weitere Hilfsstoffe zugegeben werden, wie z. B. Konservierungsmittel, Stabiliserungsmittel, Geschmackskorrigenzien und dergl.

Gewünschtenfalls können feste orale Arzneiformen auch die Freisetzung des Wirkstoffes, verzögernde Stoffe, wie z. B. Polyvinylacetate, Acrylat- oder Methacrylatcopolymere, höhere Fettalkohole und andere wachsartige Substanzen enthalten.

Die Wirkstoffe können mit den pharmazeutischen Hilfs-und/oder Trägerstoffen in an sich bekannter Weise gemischt und formuliert werden. Zur Herstellung fester Arzneiformen können die Wirkstoffe beispielsweise mit den Hilfs-und/oder Trägerstoffen in üblicher Weise gemischt und naß oder trocken granuliert werden. Je nach Art der verwendeten Zusatzstoffe kann gegebenenfalls auch durch einfaches Mischen ein direkt tablettierbares Pulver erhalten werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die Dibromverbindungen der Formel II sind in der Literatur bisher noch nicht beschrieben worden und sind ebenfalls Gegenstand der vorliegenden Erfindung. Sie stellen wertvolle Zwischenprodukte zur Herstellung der erfindungsgemäßen $N_b$-quartären Verbindungen der Formel I dar. Darüber hinaus besitzen sie, insbesondere das Dibromajmalin und -isoajmalin, selbst auch herzwirksame und blutplättchenaggregationshemmende Eigenschaften.

Erfindungsgemäß werden die Dibromverbindungen der Formel II durch Bromierung der entsprechenden Verbindungen der Formel VI.

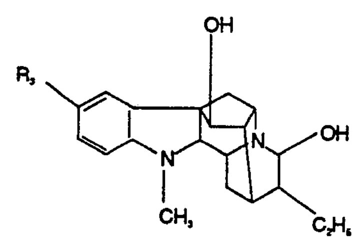

VI

worin $R_3$ Brom oder Wasserstoff bedeutet, erhalten.

Die Bromierung der Verbindungen der Formel VI erfolgt auf an sich bekannte Weise durch Umsetzung mit einem geeigneten Bromierungsmittel in einem unter den Reaktionsbedingungen inerten Lösungsmittel. Als Bromierungsmittel eignet sich insbesondere elementares Brom. Beispiele weiterer Bromierungsmittel sind N-Bromsuccinimid, Kupfer(II)bromid oder Bromchlorid. Als Lösungsmittel eignen sich Lösungsmittel, welche genügend polar sind, um die Ausgangsprodukte und die gebildeten Salze von monobromierten Zwischenprodukten in Lösung zu halten, so daß die Reaktion in einer homogenen flüssigen Phase abläuft. Beispiele geeigneter Lösungsmittel sind niedere Alkohole wie Methanol, halogenierte Kohlenwasserstoffe wie Methylenchlorid oder Chloroform, cyclische Äther wie Dioxan oder deren Gemische. Zweckmäßigerweise wird das Bromierungsmittel, vorzugsweise Brom, in einer etwa äquivalenten Menge eingesetzt, d.h. bei Verwendung von monobromierten Ausgangsprodukten wird eine ca. äquimolare Menge und bei unbromierten Ausgangsprodukten eine ca. doppelt molare Menge Brom genommen. Zur Vermeidung lokaler Überschüsse an Bromierungsmittel ist es zweckmäßig, daß Bromierungsmittel allmählich zu einer Lösung der Verbindung der Formel VI zu geben. Die Reaktionstemperatur kann zwischen –10°C und Raumtemperatur, vorzugsweise zwischen ca. –5 und ca. +5°C liegen. Dibromajmalin und Dibromsandwicin können auf an sich bekannte Weise zu den entsprechenden Isoverbindungen isomerisiert werden. Die Isomerisierung kann z. B. nach dem in der DOS 29 41 531 beschriebenen Verfahren unter alkalischen Bedingungen durch Behandeln mit einer alkoholischen Alkalimetallhydroxidlösung erfolgen.

Die nachfolgenden Beispiele sollen die Erfindung näher erläutern, ohne jedoch ihren Umfang zu beschränken. Das Verhältnis von n-Form zu Iso-Form in den erhaltenen Endprodukten wird auf an sich bekannte Weise durch NMR-Spektroskopie bestimmt.

**Beispiel 1:**

$N_b$-(3-Methylbutyl)-10,12-dibromajmalinium-jodid

1A) 100 g Ajmalin werden in einem Gemisch aus 2 l Methanol und 1,5 l Methylenchlorid gelöst. Die Lösung wird unter Rühren und Eiskühlung mit einer Lösung von 34,7 ml Brom in 200 ml Methanol versetzt. Nach 2 Stunden ist die Bromierungsreaktion beendet und das Reaktionsgemisch wird teilweise eingedampft. Das gebildete Bromierungsprodukt kristallisiert aus und wird abfiltriert. Es werden 173 g 10,12-Dibromajmalin-hydrobromid erhalten, Schmelzpunkt 226°C.

1B) Zur Freisetzung der Base werden 173 g des vorstehenden Hydrobromids in ein Gemisch aus verdünnter Natriumcarbonatlösung und Methylenchlorid gegeben und die Mischung kräftig gerührt. Anschließend wird die organische Phase abgetrennt, über Natriumsulfat getrocknet und eingedampft. Es werden 147,5 g 10,12-Dibromajmalin mit einem Schmelzpunkt von 185°C erhalten.

1C) 8g 10,12-Dibromajmalin und 6,4 ml 1-Jod-3-methylbutan werden mit 60 ml Tetramethylharnstoff versetzt und das Reaktionsgemisch 14 Tage bei Raumtemperatur gerührt. Anschließend wird durch Zugabe von 1 l Diäthyläther das gebildete Reaktionsprodukt kristallisiert und abfiltriert. Es werden 10 g $N_b$-(3-Methylbutyl)-10,12-dibromajmaliniumjodid (enthaltende ca 3 % der entsprechenden IsoajmaliniumVerbindung) mit einem Schmelzpunkt von 224°C erhalten.

**Beispiel 2:**

$N_b$-(3-Methylbutyl)-10,12-dibromajmalinium-chlorid

2A) 10 g $N_b$-(3-Methylbutyl)-10,12-dibromajmalinium-jodid werden in 200 ml eines Gemisches aus verdünnter wäßriger Natriumcarbonatlösung und Methylenchlorid gegeben und kräftig gerührt. Die organische Phase wird abgetrennt und die wäßrige Phase mehrfach mit Methylenchlorid extrahiert Die vereinigten Methylenchlorid-Phasen werden über Natriumsulfat getrocknet und teilweise eingeengt. Die erhaltene konzentrierte Lösung der der Titelverbindung entsprechenden Aldehydbase wird in eine äquimolare Menge einer verdünnten ätherischen Salzsäurelösung eingetropft. Das ausgefallene Hydrochlorid wird abfiltriert, mit Diäthyläther gewaschen und über Kaliumhydroxid unter vermindertem Druck bei 40°C getrocknet. Es werden 8,2 g $N_b$-(-3-Methylbutyl)-10,12-dibromajmalinium-chlorid (enthaltend weniger als 5 % der entsprechenden Isoajmalinium-Verbindung) mit einem Schmelzpunkt von 277°C erhalten.

**Beispiel 3:**

$N_b$-Propyl-10,12-dibromajmalinium-jodid.

5 g 10,12-Dibromajmalin werden unter Erwärmen in 150 ml Sulfolan gelöst und die Lösung mit 5 ml n-Propyljodid versetzt. Das Reaktionsgemisch wird 16 Stunden bei 80°C gerührt. Anschließend wird das ausgefallene Reaktionsprodukt abfiltriert, mit Aceton und Äther gewaschen und getrocknet. Es werden 5,2 g $N_b$-n-Propyl-10,12-dibromajmalinium-jodid (enthaltend weniger als 2 % der entsprechenden Isoajmalinium-Verbindung) erhalten, Fp >270°C (Zersetzung).

**Beispiel 4:**

$N_b$-n-Propyl-10,12-dibromajmalinium-chlorid.

2 g $N_b$-n-Propyl-10,12-dibromajmalinium-jodid werden zur Freisetzung der entsprechenden Aldehydbase in ein Gemisch aus 150 ml einer verdünnten wäßrigen Natriumcarbonat-Lösung und 150 ml Methylenchlorid gegeben und kräftig gerührt. Anschließend wird die die Aldehydbase enthaltende Methylenchloridphase abgetrennt und die wäßrige Phase noch dreimal mit je 150 ml Methylenchloridphasen werden getrocknet und eingedampft. Der Rückstand wird in 50 ml Methylenchlorid aufgenommen und mit 0,6 ml einer 5,66 normalen ätherischen Salzsäure versetzt. Die ausgefallene Titelverbindung wird abfilitriert. Es werden 1,7 g $N_b$-n-Propyl-10,12-dibromajmalinium-chlorid (enthaltend weniger als 5 % der entsprechenden Isomajmalinium-Verbindung) erhalten, Schmelzpunkt 275°C (Zersetzung).

**Beispiel 5:**

$N_b$-[2-Hydroxy-3-(1-Piperidinyl)-propyl]-10,12-dibromajmalinium-dihydrogentartrat.

10 g 10,12-Dibromajmalin und 3 g 3-Piperidinopropylenoxid werden in 75 ml Äthanol gegeben und das Reaktionsgemisch 8 Stunden bei 75°C gerührt. Anschließend werden nochmals 1 g 3-Piperidinopropylenoxid zugefügt und weitere 8 Stunden bei 75°C gerührt. Dann wird das Lösungsmittel unter vermindertem Druck abdestilliert. Der verbleibende Rückstand, welcher die der Titelverbindung entsprechende Aldehydbase enthält, wird in Aceton aufgenommen und die Lösung in eine Lösung von 3,1 g L(+)-Weinsäure in 100 ml Aceton getropft. Die ausgefallene rohe Titelverbindung wird abgesaugt. Zur weiteren Reinigung wird aus dem erhaltenen Salz nochmals die Aldehydbase durch Behandeln mit verdünnter wäßriger Natriumcarbonatlösung freigesetzt, in Aceton gelöst und die Lösung erneut in eine Lösung von 3,5 g L(+)-Weinsäure in 100 ml Aceton getropft, wobei die Titelverbindung ausfällt. Es werden 9,3 g $N_b$-[2-Hydroxy-3-(1-piperidinyl)-propyl ]-10,12-dibromajmalinium-dihydrogentartrat mit einem Schmelzpunkt von 125°C erhalten.

**Beispiel 6:**

$N_b$-n-Propyl-10,12-dibromisoajamalinium-jodid.

6A) 49,7 g 10-Bromisoajmalin werden in einer Mischung aus 1,3 l Methanol und 0,2 l Methylenchlorid gelöst und die Lösung unter Rühren und Eiskühlung mit einer Lösung von 6,9 ml Brom in 50 ml Methanol versetzt. Nach Beendigung der Bromierungsreaktion wird das Reaktionsgemisch bis zur beginnenden Kristallisation eingedampft und das ausgefallene 10,12-Dibromisoajmalin-hydrobromid wird abfiltriert. Ausbeute 65,8 g, Schmelzpunkt 215 - 218°C.
10,12-Dribromisoajmalin-hydrobromid kann auch durch Bromierung von Isoajmalin nach der in Beispiel 1A beschriebenen Methode erhalten werden, indem man 25 g Isoajmalin in 1 l Methanol unter Zusatz von Methylenchlorid löst und die Lösung unter Rühren und Eiskühlung mit einer Lösung von 8,4 ml Brom in 50 ml Methanol versetzt. Nach Aufarbeitung des Reaktionsgemisches werden 42 g 10,12-Dibromisoajmalin-hydrobromid, Schmelzpunkt 215 - 218°C, erhalten.

6B) Aus 65 g 10,12-Dibromisoajmalin-hydrobromid wird nach der in Beispiel 1B beschriebenen Methode die Base freigesetzt. Es werden 48,1 g 10,12-Dibromisoajmalin erhalten, Schmelzpunkt 186°C.
10,12-Dibromisoajmalin kann aus durch Isomerisierung von 10,12-Dibromajmalin erhalten werden. Hierzu werden 2 g 10,12-Dibromajmalin und 1,4 g Kaliumhydroxid in 20 ml Methanol gelöst und die Lösung 6 Stunden unter Rückfluß erhitzt. Anschließend wird das Reaktionsgemisch etwas eingeengt, mit Wasser verdünnt und mit Methylenchlorid extrahiert. Die Methylenchloridphase wird abgetrennt, getrocknet und zur Trockne eingedampft. Der verbleibende Rückstand wird aus Methanol kristallisiert. Es werden 1,3 g 10,12-Dibromisoajmalin, Schmelzpunkt 186°C, erhalten.

6C) 7 g 10,12-Dibromisoajmalin werden unter Erwärmen in 250 ml Acetonitril gelöst. Die Lösung wird mit 7 ml n-Propyljodid versetzt und 5 Stunden unter Rückfluß erhitzt. Anschließend wird die gebildete Titelverbindung abfiltriert, mit Acetonitril gewaschen und getrocknet. Es werden 8,1 g $N_b$-n-Propyl-10,12-dibromisoajmalinium-jodid (enthaltend ca. 10 % der entsprechenden Ajmalinium-Verbindung) erhalten. Schmelzpunkt 240°C (Zersetzung).

**Beispiel 7:**

$N_b$-n-Propyl-10,12-dibromisoajmalinium-hydrogentartrat.

11,7 g $N_b$-n-Propyl-10,12-dibromisoajmalinium-jodid werden in ein Gemisch aus 100 ml 10 %-iger wäßriger Natriumcarbonatlösung und 250 ml Essigsäureäthylester gegeben und das Reaktionsgemisch kräftig gerührt. Anschließend wird die organische Phase, welche die der Titelverbindung entsprechende Aldehydbase enthält, abgetrennt, die

wäßrige Phase mehrfach mit Essigsäureäthylester extrahiert, und die vereinigten organischen Phasen über Natriumsulfat getrocknet und auf ein Volumen von ca. 300 ml eingeengt. Die so erhaltene Lösung der Aldehydbase wird in eine mit 1 l Essigsäureäthylester verdünnte Lösung von 1,7 g L(+)-Weinsäure in wenig Aceton eingetropft. Zur vollständigen Ausfällung der Titelverbindung wird das Reaktionsgemisch 12 Stunden im Kühlschrank bei ca. 5°C stehengelassen. Anschließend wird die ausgefällte Titelverbindung abfiltriert. Es werden 6,1 g $N_b$-n-Propyl-10,12-dibromisoajmalinium-hydrogentartrat (enthaltend ca. 10 % der entsprechenden Ajmalinium-Verbindung) mit einem Schmelzpunkt von 140 - 145°C erhalten.

**Beispiel 8:**

$N_b$-(3-Morpholinopropyl)-10,12-dibromisoajmalinium-chlorid.

9,26 g N-(3-Chlorpropyl)-morpholin-hydrochlorid werden zur Freisetzung der Base mit 20 ml 10 %-iger wäßriger Natriumcarbonatlösung und 50 ml Diäthyläther versetzt. Die ätherische Phase wird abgetrennt, über Natriumsulfat getrocknet, unter leicht vermindertem Druck auf ein Volumen von ca. 20 ml eingeengt und in eine Lösung von 15 g 10,12-Dibromajmalin in 300 ml Äthanol eingetropft. Das Reaktionsgemisch wird 20 Stunden am Rückfluß erhitzt und anschließend eingedampft. Der Rückstand wird mit Isopropanol aufgenommen. Die erhaltene Lösung wird bis zur beginnenden Kristallisation der Titelverbindung eingeengt und anschließend zur Vervollständigung der Ausfällung 12 Stunden bei 5°C stehengelassen. Es werden 12,5 g $N_b$-(3-Morpholinopropyl)-10,12-dibromisoajmalinium-chlorid (enthaltend ca. 25 % der entsprechenden Ajmalinium-Verbindung) erhalten. Schmelzpunkt 204°C.

**Beispiel 9:**

$N_b$-Methyl-10,12-dibromsandwicinium-jodid.

*9A)* 40 g Sandwicin werden in einem Gemisch aus 1,3 l Methanol und 0,2 l Methylenchlorid gelöst und unter Rühren und Eiskühlung mit einer Lösung von 14 ml Brom in 100 ml Methanol verletzt. Nach vollständiger Zugabe läßt man noch 30 Minuten rühren und tropft sodann zu der 10,12-Dibromsandwicin-hydrobromid enthaltenden Lösung in der Kälte wäßrige Natriumcarbonatlösung zu, bis die aus dem Salz freigesetzte Base ausfällt. Die ausgefallene Base wird abgetrennt und mit Wasser und Aceton gewaschen. Es werden 57,4 g 10,12-Dibromsandwicin, Schmelzpunkt 187 - 190°C, erhalten.
Gewünchtenfalls kann das 10,12-Dibromsandwicin in sein Hydrogenfumarat überführt werden. Hierzu werden 6 g 10,12-Dibromsandwicin und 1,45 g Fumarsäure in Methanol gelöst und die Lösung bis zur Trockne eingedampft. Es werden 7,45 g 10,12-Dibromsandwicin-hydrogenfumarat erhalten, Schmelzpunkt 175°C.

*9B)* 10 g 10,12-dibromsandwicin und 11 ml Jodmethan werden in 500 ml Acetonitril gegeben und das Reaktionsgemisch 8 Stunden am Rückfluß erhitzt. Die nach Abkühlen des Reaktionsgemisches ausgefallene Titelverbindung wird abfiltriert. Es werden 11,2 g $N_b$-Methyl-10,12-Dibromsandwiciniumjodid (enthaltend ca. 5 % der entsprechenden Isosandwicinium-Verbindung) mit einem Schmelzpunkt von 242°C erhalten.

**Beispiel 10:**

$N_b$-Methyl-10,12-Dibromsandwicinium-hydrogentartrat.

11,2 g $N_b$-Methyl-10,12-dibromsandwicinium-jodid werden nach der in Beispiel 7 angegebenen Vorschrift zunächst durch Behandlung mit einem Gemisch aus 10 %-iger wäßriger Natriumcarbonatlösung und Essigsäureäthylester in die entsprechende Aldehydbase überführt und die erhaltenen Lösung der Aldehydbase unter Rühren mit einer Lösung einer äquimolaren Menge L-(+)-Weinsäure in Aceton versetzt. Die Titelverbindung fällt aus und wird abfiltriert. Es werden 11,1 g $N_b$-Methyl-10,12-dibromsandwicinium-hydrogentartrat (enthaltend weniger als 5 % der entsprechenden Isosandwicinium-Verbindung) mit einem Schmelzpunkt von 155 - 158°C erhalten.

**Beispiel 11:**

$N_b$-Methyl-10,12-dibromisosandwicinium-jodid.

*11A)* 6,6 g Isosandwicin werden in einem Gemisch aus 100 ml Methylenchlorid und 100 ml Methanol gelöst und zu der Lösung unter Rühren und Eiskühlung innerhalb einer Stunde eine Lösung von 6,6 g Brom in Methylenchlorid zugesetzt. Nach weiteren 30 Minuten wird das Reaktionsgemisch teilweise eingeengt, wobei das gebildete 10,12-Dibromisosandwicin-hydrobromid ausfällt. Ausbeute 8,6 g, Schmelzpunkt 216°C.

*11B)* Aus 10 g 10,12-Dibromisosandwicin-hydrobromid wird die Base nach der in Beispiel 1B beschriebenen Methode freigesetzt. ES werden 8 g 10,12-Dibromisosandwicin, Schmelzpunkt 179 - 182°C, erhalten.

10,12-Dibromisosandwicin kann auch durch Isomerisierung von 10,12-Dibromsandwicin erhalten werden. Hierzu werden 11 g 10,12-Dibromsandwicin und 10 g Kaliumhydroxid in 180 ml Methanol gelöst und die Lösung 15 Stunden unter Rückfluß erhitzt. Anschließend wird mit Wasser versetzt und mit Methylenchlorid extrahiert. Die Methylenchloridphase wird getrocknet und bis zur beginnenden Kristallisation eingeengt. Das auskristallisierte 10,12-Dibromisosandwicin wird abfiltriert. Ausbeute 9,5 g, Schmelzpunkt 179 - 182°C. Gewünschtenfalls kann das 10,12-Dibromisosandwicin aus einem Gemisch von Methylenchlorid und Methanol umkristallisiert werden.

Gewünschtenfalls kann das 10,12-Dibromisosandwicin durch Umsetzung mit Fumarsäure in sein Hydrogenfumarat überführt werden. Hierzu werden 5,4 g 10,12-Dibromisosandwicin und 1,3 g Fumarsäure in Methanol gelöst. Die Lösung wird etwas eingeengt, mit Diäthyläther versetzt und auf 5°C abgekühlt. Das ausfallende 10,12-Dibromisosandwicin-hydrogenfumarat wird abfiltriert. Ausbeute 5,4 g, Schmelzpunkt 180°C.

11C) 5,7 g 10,12-Dibromisosandwicin werden unter Erwärmen in 250 ml Acetonitril gegeben. Anschließend werden 6 ml Methyljodid zugegeben und das Reaktionsgemisch 8 Stunden am Rückfluß erhitzt. Dann wird die Lösung eingeengt und der Rückstand mit Aceton aufgenommen. Die gebildete Titelverbindung fällt aus und wird abfiltriert. Es werden 5,2 g $N_b$-Methyl-10,12-dibromisosandwicinium-jodid (enthaltend weniger als 5 % der entsprechenden Sandwicinium-Verbindung) erhalten, Schmelzpunkt 255°C (Zersetzung).

**Beispiel 12:**

$N_b$-Methyl-10,12-dibromisosandwicinium-hydrogenfumarat.

5g $N_b$-Methyl-dibromisosandwicinium-jodid werden in 250 ml wäßrige 10 %-ige Natriumcarbonatlösung gegeben und das Reaktionsgemisch dreimal mit je 250 ml Essigsäure äthylester extrahiert. Die Essigsäureäthylesterphasen werden vereinigt und getrocknet und die so erhaltene Lösung der der Titelverbindung entsprechenden Aldehydbase wird bis fast zur Trockne eingeengt, der Rückstand in wenig Methanol gelöst, die Lösung mit 0,93 g Fumarsäure versetzt und bis zur beginnenden Kristallisation eingeengt. Zur Vervollständigung der Fällung wird das Reaktionsgemisch 12 Stunden im Kühlschrank bei ca. 5°C stehengelassen. Anschließend wird die gebildete Titelverbindung abfiltriert. Es werden 4,5 g $N_b$-Methyl-10,12-dibromisosandwicinium-hydrogenfumarat (enthaltend weniger als 5 % der entsprechenden Sandwicinium-Verbindung) mit einem Schmelzpunkt von 256 - 258°C erhalten.

Nach den in den vorstehenden Beispielen 1 - 12 beschriebenen Verfahren können auch die in der folgenden Tabelle aufgeführten Verbindungen der Formel I hergestellt werden. In der Tabelle werden die Verbindungen durch Angabe des Strukturtyps (Aj = Ajmalin, Isoaj = Isoajmalin, Sa = Sandwicin, Isosa = Isosandwicin) und gegebenenfalls des Mischungsverhältnisses zwischen zwei Epimeren gekennzeichnet.

| Bsp. Nr | Strukturtyp | R | A | Fp. °C |
|---|---|---|---|---|
| 13 | Aj* | $CH_3$ | Cl | |
| 14 | Aj* | $CH_3$ | HT | 140 |
| 15 | Isoaj* | $CH_3$ | J | 268(Z) |
| 16 | Isoaj* | $CH_3$ | HT | 165 - 173 |
| 17 | Isoaj* | $CH_3$ | Cl | 285(Z) |
| 18 | Aj | $C_2H_5$ | J | |
| 19 | Aj* | $CH_3$ | Me | 236 - 238 |
| 20 | Aj* | $CH_3$ | J | 245 - 250 |
| 21 | Aj* | n-$C_3H_7$ | HT | 192 |
| 22 | Isoaj* | n-$C_3H_7$ | HT | 210 |
| 23 | SA* | n-$C_3H_7$ | J | 260(Z) |
| 24 | SA* | n-$C_3H_7$ | HT | 153 - 154 |
| 25 | Aj** | n-$C_4H_9$ | J | 255(Z) |
| 26 | Aj/Isoaj 9 : 1 | n-$C_4H_9$ | HFu | 165 - 170 |
| 27 | Isosa* | n-$C_4H_9$ | J | 255 |
| 28 | Isosa* | n-$C_4H_9$ | HFu | 151 |
| 29 | Aj* | n-$C_5H_{11}$ | J | 243 |
| 30 | Aj* | n-$C_5H_{11}$ | Cl | 245 |
| 31 | Isoaj* | n-$C_5H_{11}$ | Br | 254 |
| 32 | Isoaj* | n-$C_5H_{11}$ | J | |
| 33 | Isoaj 9 : 1 | n-$C_5H_{11}$ | HFu | 154 |
| 34 | Aj/Isoaj 2 : 1 | n-$C_6H_{11}$ | J | |
| 35 | Aj/Isoaj 2 : 1 | n-$C_6H_{13}$ | HT | 120 |
| 36 | Sa** | n-$C_6H_{13}$ | J | 231 |
| 37 | Isosa* | n-$C_6H_{13}$ | HT | 131 - 132 |
| 38 | Aj/Isoaj 3 : 7 | $(CH_3)_2CH-CH_2-CH_2$ | HT | 158 |
| 39 | Isoaj | $(CH_3)_2CH-CH_2-CH_2$ | Cl | 260 - 265 |

| | | | | |
|---|---|---|---|---|
| 40 | Sa/Isosa 9 : 1 | $(CH_3)_2CH-CH_2-CH_2$ | J | 260 |
| 41 | Sa | $(CH_3)_2CH-CH_2-CH_2$ | Cl | 251 |
| 42 | Isosa* | $(CH_3)_2CH-CH_2-CH_2$ | HFu | 166 - 167 |
| 43 | Isoaj* | $CH_2=CH-CH_2$ | Br | 275 - 285 |
| 44 | Isoaj | $CH=CH-CH_2$ | HT | 145 - 147 |
| 45 | Sa* | $CH_2=CH-CH_2$ | Br | 257 - 258 |
| 46 | Sa/Isosa > 9 : < 1 | $CH_2=CH-CH_2$ | HT | 147 - 149 |
| 47 | Aj* | Phen-$(CH_2)_3$ | Br | 251 |
| 48 | Aj* | Phen-$(CH_2)_3$ | HFu | 148 |
| 49 | Aj** | $(CH_3)_2N-CH_2-CH_2$ | Cl | |
| 50 | Aj/Isoaj 1 : 2 | $(CH_3)_2N-CH_2-CH_2$ | di-HFu | 124 |
| 51 | Isoaj* | $(C_2H_5)_2N-CH_2-CH_2$ | Cl | 201 |
| 52 | Aj/Isoaj 2 : 3 | $(C_3H_5)_2N-CH_2-CH_2$ | di-HT | 107 - 110 |
| 53 | Sa/Isosa 4 : 6 | $(C_2H_5)_2N-CH_2-CH_2$ | Cl | |
| 54 | Sa/Isosa 33 : 67 | $(C_2H_5)_2N-CH_2-CH_2$ | di-HT | 214 - 215 |
| 55 | Aj/Isosa 15 : 85 | $[(CH_3)_2CH]_2N-CH_2-CH_2$ | Cl | 184 |
| 56 | Aj** | Pyrr-$CH_2-CH_2$ | Cl | |
| 57 | Aj/Isoaj 2 : 8 | Pyrr-$CH_2-CH_2$ | HT | 132 |
| 58 | Sa/Isosa 33 : 67 | Pyrr-$CH_2-CH_2$ | Cl | 164 - 168 |
| 59 | Sa/Isosa 1 : 9 | Pyrr-$CH_2-CH_2$ | di-HFu | 116 |
| 60 | Isoaj* | Pip-$CH_2-CH_2$ | Cl | 230 |
| 61 | Isoaj* | Az-$CH_2-CH_2$ | Cl | 170 |
| 62 | Isoaj* | Morph-$CH_2-CH_2$ | Cl | 200 - 202 |
| 63 | Aj/Isoaj 1 : 9 | Morph-$CH_2-CH_2$ | di-HT | 120 - 130 |
| 64 | Sa** | Morph-$CH_2-CH_2$ | Cl | |
| 65 | Sa/Isosa 2 : 3 | Morph-$CH_2-CH_2$ | di-HT | 117 - 118 |
| 66 | Aj/Isoaj 2 : 3 | $(C_2H_5)_2N-(CH_2)_3$ | Cl | 146 |
| 67 | Aj/Isoaj 1 : 1 | $(C_2H_5)_2N-(CH_2)_3$ | di-HT | 115 |
| 68 | Sa/Isosa 1 : 1 | $(C_2H_5)_2N-(CH_2)_3$ | Cl | 200 |
| 69 | Aj/Isoaj 1 : 1, 8 | Pip-$(CH_2)_3$ | di-HFu | 120 |
| 70 | Sa** | (Pyr-2)-$CH_2-CH_2$ | Cl | |
| 71 | Sa/Isosa 1 : 2 | (Pyr-2)-$CH_2-CH_2$ | di-HFu | 138 |
| 72 | Sa** | Pip-$CH_2$-CH-$CH_2$<br>\|<br>OH | di-HT | 136 - 138 |
| 73 | Isoaj* | HO-$CH_2-CH_2$ | Cl | 265 - 270 |
| 74 | Aj** | $CH_3$-CHOH-$CH_2$ | HT | |

* enthaltend weniger als 5 % des entsprechenden Iso- oder n-Epimeren,
** Epimerengehalt nicht bestimmt,

| | | | |
|---|---|---|---|
| J = Jodid | Cl = Chlorid | Br = Bromid | HT = Hydrogentartrat |
| HFu = Hydrogenfumarat | ME = Methylsulfonat | Z = unter Zersetzung | Morph = Morpholin-1-yl |
| Pyrr = Pyrrolidin-1-yl | Pyr-2 = 1-Methylpyrrolidin-2-yl | Pip = Piperidin-1-yl | Az = Azepin-1-yl. |

**Beispiel I**

Tabletten enthaltend
$N_b$-Propyl-10,12-dibromajmalinium-hydrogentartrat

Zusammensetzung:

| | | |
|---|---|---|
| $N_b$-Propyl-10,12-dibromajmalinium-hydrogentartrat | 15 | Teile |
| Lactose | 30 | " |
| Weizenstärke | 55 | Teile |
| Gelatine | 1 | Teil |
| hochdisperse Kieselsäure (Aerosil$^R$ 200) | 2 | Teile |
| hydriertes Rhizinusöl | 2 | " |
| insgesamt | 105 | Teile |

Der Wirkstoff wird mit Lactose und Stärke vermischt, die entstandene Mischung wird mit einer 15 %-igen wäßrigen Lösung der Gelatine durchfeuchtet und granuliert. Die feuchte Masse wird durch ein 1,6 mm-Sieb passiert, bei 35°C auf Horden getrocknet und anschließend durch ein 1,0 mm-Sieb passiert. Nach dem Vermischen des Granulates mit Aerosil® 200 und dem gepulvertem hydriertem Rhizinusöl werden aus dem Gemisch Tabletten von 105 mg Gewicht gepreßt, so daß jede Tablette 15 mg Wirkstoff enthält.

**Beispiel II**

Kapseln enthaltend $N_b$-[(Diäthylamino)propyl]-10,12-dibromajmaliniumhydrochlorid und $N_b$-[(Diäthylamino)propyl]10,12-dibromisoajmalinium-hydrochlorid.

Zusammensetzung:

| | |
|---|---|
| Gemisch $N_b$-[(Diäthylamino)propyl]-10,12-dibromajmalinium-hydrochlorid/$N_b$-[(Diäthylamino)-propyl]-10,12-dibromisoajmalinium-hydrochlorid 2 : 3 | 20 Teile |
| Lactose | 65 " |
| Mikrokristalline Cellulose | 30 " |
| Magnesiumstearat | 1 Teil |
| Total | 116 Teile |

Herstellungsvorschrift:

Der Wirkstoff wird mit Lactose, mikrokristalliner Cellulose und Magnesiumstearat gründlich vermischt. Diese Pulvermischung wird automatisch so in Hartgelatinekapseln der Größe 4 abgefüllt, daß jede Kapsel 15 mg Wirkstoff enthält.

**Patentansprüche**

1. $N_b$-quartäre Verbindungen der allgemeinen Formel I

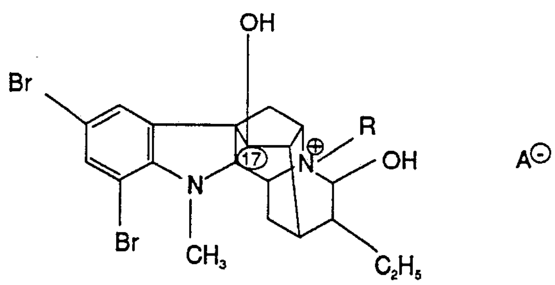

I

worin

R für eine Alkylgruppe mit 1 - 6 Kohlenstoffatomen steht, welche gegebenenfalls an einem nicht an Stickstoff gebundenen Kohlenstoffatom substituiert sein kann durch Hydroxy oder Halogen, für eine Alkenylgruppe mit 3 bis 6 Kohlenstoffatomen, deren Doppelbindung nicht direkt an das Stickstoffatom gebunden ist, für eine Phenylalkylgruppe mit 2 bis 3 Kohlenstoffatomen in der Alkylenkette, welche gegebenenfalls im Phenylring durch Alkoxy mit 1 - 3 Kohlenstoffatomen oder Halogen substituiert sein kann, oder für eine Gruppe a steht,

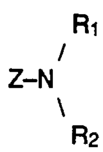

a

worin

| | |
|---|---|
| Z | eine Alkylenkette mit 2 - 3 Kohlenstoffatomen oder die 2-Hydroxypropylenkette bedeutet, |
| $R_1$ und $R_2$ | je Alkyl mit 1 - 3 Kohlenstoffatomen bedeuten oder |
| $R_1$ und $R_2$ | zusammen mit dem Stickstoffatom, an das sie gebunden sind, einen Heterocyclus aus der Gruppe Pyrrolidin, Piperidin, Azepin und Morpholin darstellen, oder falls Z eine Alkylenkette ist, |
| $R_1$ | Alkyl mit 1 - 3 Kohlenstoffatomen bedeutet und $R_2$ zusammen mit dem Stickstoffatom, an welches es gebunden ist, und dem diesem Stickstoffatom benachbarten C-Atom der Alkylenkette Z einen Heterocyclus aus der Gruppe Pyrrolidin und Piperidin darstellt, und |

$A^-$ das Anion einer physiologisch verträglichen anorganischen oder organischen Säure bedeutet.

2. $N_b$-quartäre Verbindungen gemäß Anspruch 1, dadurch gekennzeichnet, daß R für eine Alkylgruppe mit 1 - 5 Kohlenstoffatomen oder eine Gruppe a steht und $A^-$ die in Anspruch 1 genannte Bedeutung besitzt.

3. $N_b$-quartäre Verbindungen gemäß Anspruch 2, dadurch gekennzeichnet, daß R eine Alkylgruppe mit 2 - 5 Kohlenstoffatomen oder die Gruppe a darstellt, worin Z die in Anspruch 2 angegebene Bedeutung besitzt, $R_1$ und $R_2$ je Alkyl mit 1 - 2 Kohlenstoffatomen bedeuten oder zusammen mit dem Stickstoffatom, an welches sie gebunden sind, einen Pyrrolidin-, Piperidin-, Azepin- oder Morpholinring darstellen und $A^-$ die in Anspruch 2 angegebene Bedeutung besitzt.

4. $N_b$-quartäre Verbindungen gemäß Anspruch 2, dadurch gekennzeichnet, daß sie $N_b$-quartäre Dibromajmalin- und/oder Dibromisoajmalinderivate der allgemeinen Formel I, worin $C_{17}$ R-konfiguriert vorliegt und $A^-$ und R die in Anspruch 2 genannte Bedeutung besitzen, darstellen.

5. $N_b$-quartäre Verbindungen gemäß Anspruch 4, dadurch gekennzeichnet, daß R n-Propyl, 3-Methylbutyl, n-Pentyl, Diäthylaminopropyl oder Morpholinoäthyl darstellt und $A^-$ die in Anspruch 4 genannte Bedeutung besitzt.

6. Arzneimittel, dadurch gekennzeichnet, daß sie neben üblichen pharmazeutischen Hilfs- und/oder Trägerstoffen eine pharmakologisch wirksame Menge einer oder mehrerer $N_b$-quartärer Verbindungen der Formel I enthalten.

7. Verbindungen der allgemeinen Formel II

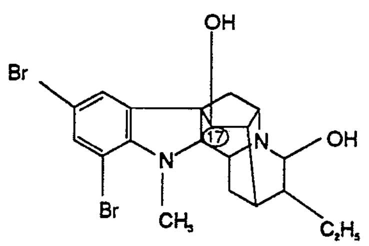

II

8. Verfahren zur Herstellung von $N_b$-quartären Verbindungen der allgemeinen Formel I

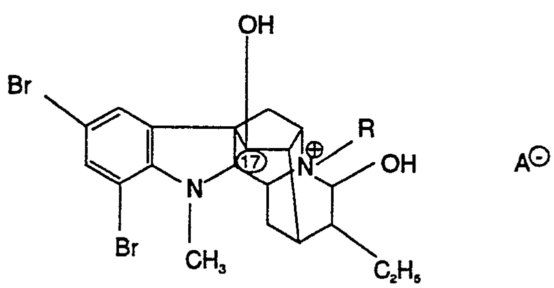

I

worin

R und A die in Anspruch 1 angegebene Bedeutung besitzen, dadurch gekennzeichnet, daß man Verbindungen der allgemeinen Formel II

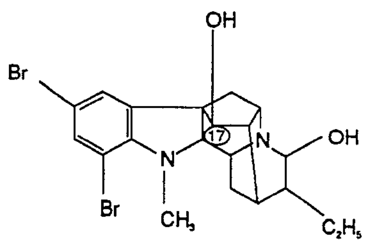

II

mit Verbindungen der allgemeinen Formel III

R – X　　III

worin R obige Bedeutung besitzt und X einen aminolytisch abspaltbaren Rest bedeutet, umsetzt.

9. Verfahren zur Herstellung von Verbindungen der allgemeinen Formel I'

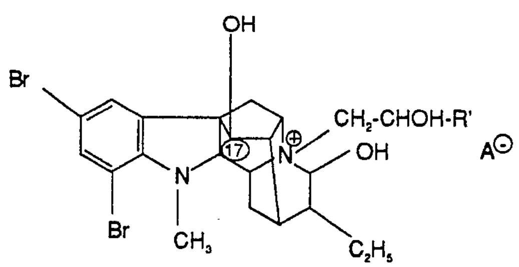

I'

worin $A^-$ die in Anspruch 1 angegebene Bedeutung besitzt und R' Wasserstoff, Alkyl mit 1 - 4 Kohlenstoffatomen oder eine $CH_2NR_1R_2$-Gruppe bedeutet, worin $R_1$ und $R_2$ die in Anspruch 1 angegebene Bedeutung besitzen, Verbindungen der allgemeinen Formel II mit Epoxiden der allgemeinen Formel IV

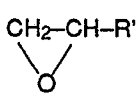

IV

worin R' obige Bedeutung besitzt, umsetzt zu Verbindungen der allgemeinen Formel Va

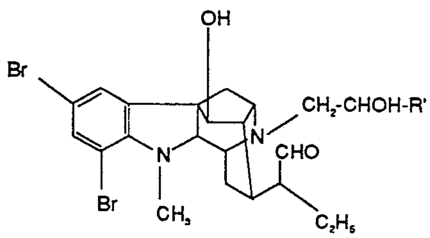

Va

worin R' obige Bedeutung besitzt, und diese anschließend durch Behandlung mit Säuren der Formel HA, worin A obige Bedeutung besitzt, in $N_b$-quartäre Verbindungen der allgemeinen Formel I' überführt.

10. Verfahren zur Herstellung von Verbindungen der allgemeinen Formel II

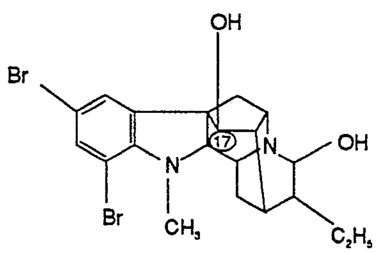

II

dadurch gekennzeichnet, daß man Verbindungen der allgemeinen Formel VI

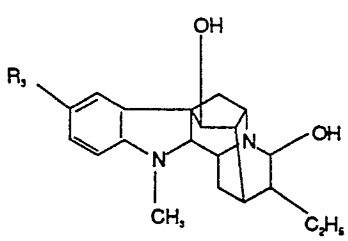

VI

worin $R_3$, Brom oder Wasserstoff ist, bromiert.

## Claims

1. $N_b$-quaternary compounds of the general Formula I,

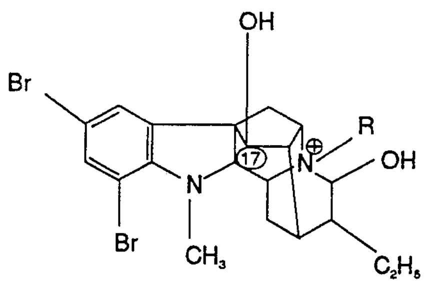

$A^{\ominus}$

I

wherein

R stands for an alkyl group with 1 - 6 carbon atoms, which may optionally be substituted by hydroxy or halogen on a carbon atom which is not bonded to nitrogen, for an alkenyl group with 3 to 6 carbon atoms, the double bond of which is not directly bonded to the nitrogen atom, for a phenylalkyl group with 2 to 3 carbon atoms in the alkylene chain, which may optionally be substituted in the phenyl ring by alkoxy with 1 - 3 carbon atoms or halogen, or stands for a group a,

```
      R1
     /
Z–N
     \
      R2
```

a

wherein

Z is an alkylene chain with 2 - 3 carbon atoms or the 2-hydroxypropylene chain,
$R_1$ and $R_2$ are each alkyl with 1 - 3 carbon atoms, or
$R_1$ and $R_2$, together with the nitrogen atom to which they are bonded, represent a heterocycle from the group pyrrolidine, piperidine, azepine and morpholine, or if Z is an alkylene chain,
$R_1$ is alkyl with 1 - 3 carbon atoms and $R_2$, together with the nitrogen atom to which it is bonded and the C atom of the alkylene chain Z which adjoins said nitrogen atom, represents a heterocycle from the group pyrrolidine and piperidine, and
$A^-$ is the anion of a physiologically compatible inorganic or organic acid.

2. $N_b$-quaternary compounds according to Claim 1, characterised in that R stands for an alkyl group with 1 - 5 carbon atoms or a group *a*, and $A^-$ has the meaning given in claim 1.

3. $N_b$-quaternary compounds according to Claim 2, characterised in that R represents an alkyl group with 2 - 5 carbon atoms or the group a, wherein Z has the meaning given in Claim 2, $R_1$ and $R_2$ are each alkyl with 1 - 2 carbon atoms or, together with the nitrogen atom to which they are bonded, represent a pyrrolidine, piperidine, azepine or morpholine ring, and A has the meaning given in Claim 2.

4. $N_b$-quaternary compounds according to Claim 2, characterised in that they represent $N_b$-quaternary dibromoajmaline and/or dibromoisoajmaline derivatives of the general Formula I, wherein $C_{17}$ is present in the R-configuration and $A^-$ and R have the meanings given in Claim 2.

5. $N_b$-quaternary compounds according to Claim 4, characterised in that R represents n-propyl, 3-methylbutyl, n-pentyl, diethylaminopropyl or morpholinoethyl, and $A^-$ has the meaning given in Claim 4.

6. Medicaments, characterised in that they contain a pharmacologically effective quantity of one or more $N_b$-quaternary

compounds of Formula I as well as conventional pharmaceutical auxiliaries and/or carriers.

7. Compounds of the general Formula II

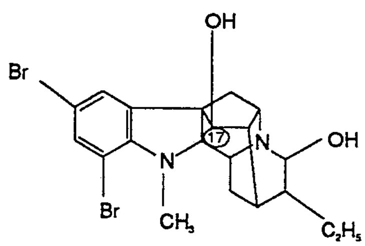

II

8. Method for producing Nb-quaternary compounds of the general Formula I,

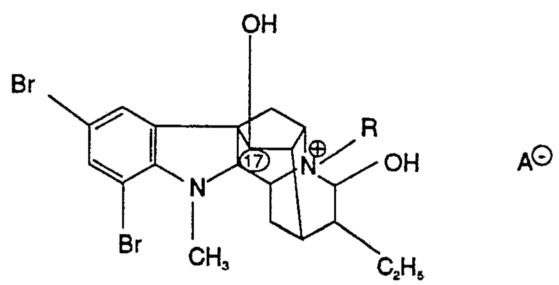

I

wherein

R and A have the meanings given in Claim 1, characterised in that compounds of the general Formula II,

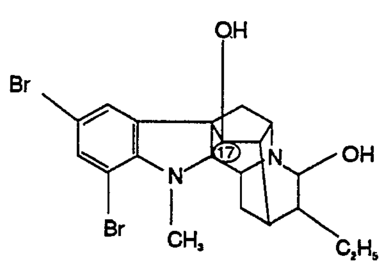

II

are reacted with compounds of the general Formula III,

R - X III

wherein R has the above meaning and X is an aminolytically cleavable radical.

9. Method for producing compounds of the general Formula I',

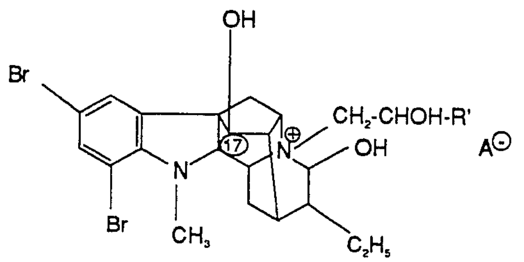

I'

wherein $A^-$ has the meaning given in claim 1 and R' is hydrogen, alkyl with 1 - 4 carbon atoms or a $CH_2NR_1R_2$ group, wherein $R_1$ and $R_2$ have the meanings given in Claim 1, characterised in that compounds of the general Formula II are reacted with epoxides of the general Formula IV,

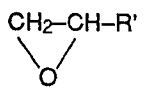

IV

wherein R' has the above meaning, to produce compounds of the general Formula Va,

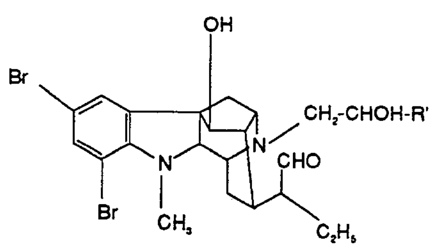

Va

wherein R' has the above meaning, and these are then converted into $N_b$-quaternary compounds of the general Formula I' by treatment with acids of the formula HA, wherein A has the above meaning.

10. Method for producing compounds of the general Formula II,

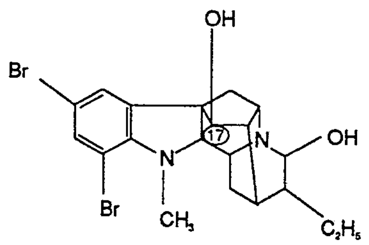

II

characterised in that compounds of the general Formula VI,

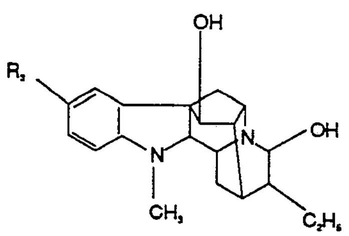

VI

wherein $R_3$ is bromine or hydrogen, are brominated.

**Revendications**

1. Des composés $N_b$-quaternaires de formule générale I

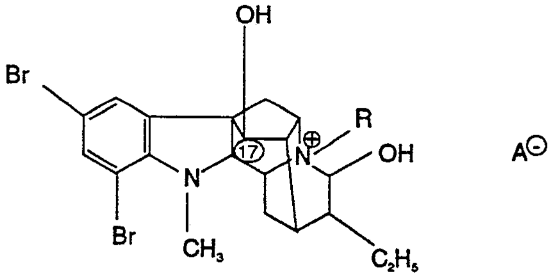

I

dans laquelle

R Représente un groupe alkyle avec 1 à 6 atomes de carbone qui peut éventuellement être substitué par un hydroxyle ou un halogène sur un atome de carbone non lié à l'azote, un groupe alcényle avec 3 à 6 atomes de carbone dont la double liaison n'est pas directement liée à l'atome d'azote, un groupe phénylalkyle avec 2 à'3 atomes de carbone dans la chaîne alkylène, qui peut éventuellement être substitué dans le noyau phényle par un alcoxy ayant 1 à 3 atomes de carbone ou par un halogène ou représente un groupe a

```
      R1
     /
Z–N
     \
      R2
```

a

où

Z signifie une chaîne alkylène avec 2 à 3 atomes de carbone ou la chaîne 2-hydroxypropylène.
$R_1$ et $R_2$ signifient chacun un alkyle avec 1 à 3 atomes de carbone ou
$R_1$ et $R_2$ représentent, ensemble avec l'atome d'azote auquel ils sont liés, un hétérocycle du groupe des pyrrolidine, pipéridine, azépine et morpholine ou, dans le cas où Z est une chaîne alkylène,
$R_1$ signifie un alkyle avec 1 à 3 atomes de carbone et $R_2$ représente, ensemble avec l'atome d'azote auquel il est lié et l'atome de C de la chaîne alkylène voisin de cet atome d'azote, un hétérocycle du groupe des pyrrolidine et pipéridine, et
$A^-$ signifie l'anion d'un acide minéral ou organique physiologiquement compatible.

2. Composés $N_b$ -quaternaires selon la revendication 1, caractérisés en ce que R représente un groupe alkyle avec 1 à 5 atomes de carbone ou un groupe a et que $A^-$ a la signification indiquée dans la revendication 1.

3. Composés $N_b$-quaternaires selon la revendication 2, caractérisés en ce que R représente un groupe alkyle avec 2 à 5 atomes de carbone ou le groupe a, où Z a la signification indiquée dans la revendication 2, que $R_1$ et $R_2$ signifient chacun un alkyle avec 1 à 2 atomes de carbone ou représentent, ensemble avec l'atome d'azote auquel ils sont liés, un noyau de pyrrolidine, de pipéridine, d'azépine ou de morpholine et que $A^-$ possède la signification indiquée dans la revendication 2.

4. Composés $N_b$-quaternaires selon la revendication 2, caractérisés en ce qu'ils représentent des dérivés $N_b$-quaternaires de dibromo-ajmaline et/ou de dibromo-isoajmaline de formule générale I dans laquelle $C_1$ est présent en la configuration R et $A^-$ et R possèdent la signification indiquée dans la revendication 2.

5. Composés $N_b$-quaternaires selon la revendication 4, caractérisés en ce que R représente un n-propyle, un 3-méthylbutyle, un n-pentyle, un diéthylaminopropyle ou un morpholinoéthyle et $A^-$ possède la signification indiquée dans la revendication 1.

6. Médicaments, caractérisés en ce qu'ils contiennent, à côté des adjuvants et/ou excipients usuels, une quantité pharmacologiquement efficace d'un ou plusieurs composés $N_b$-quaternaires de formule I.

7. Composés de formule générale II

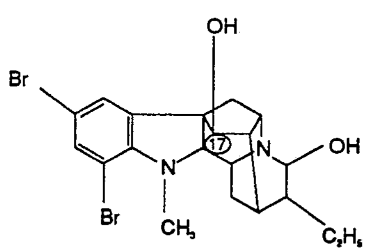

III

8. Procédé de préparation de composés $N_b$-quaternaires de formule I

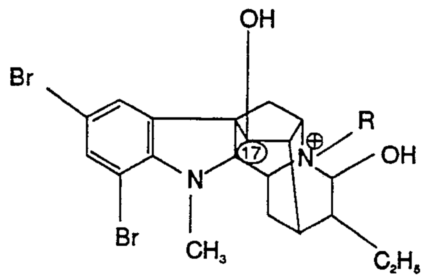

A⊖

I

dans laquelle

R et A ont la signification indiquée dans la revendication 1, caractérisés en ce que l'on fait réagir des composés de formule générale II

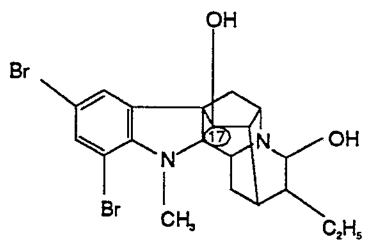

II

avec des composés de formule générale III

R - X III

dans laquelle R possède la signification précédente et X signifie un reste éliminable par aminolyse.

9. Procédé de préparation de composés de formule générale I'

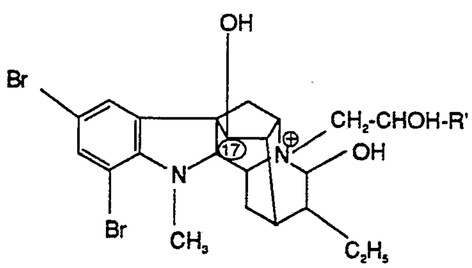

A⊖

I'

dans laquelle A possède la signification indiquée dans la revendication 1 et R' signifie l'hydrogène, un alkyle avec 1 à 4 atomes de carbone ou un groupe $CH_2NR_1R_2$ où $R_1$ et $R_2$ possèdent les significations indiquées dans la revendication 1, caractérisé en ce que l'on fait réagir des composés de formule générale II avec des époxydes de formule générale IV

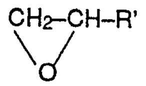

IV

dans laquelle R' a la signification précédente, en composés de formule générale Va

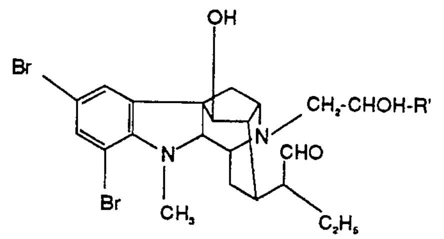

Va

dans laquelle R' a la signification ci-dessus et que l'on transforme ceux-ci subséquemment en composés $N_b$-quaternaires de formule générale I' par traitement à l'aide d'acides de formule HA où A possède la signification précédente.

10. Procédé de préparation de composés de formule générale II

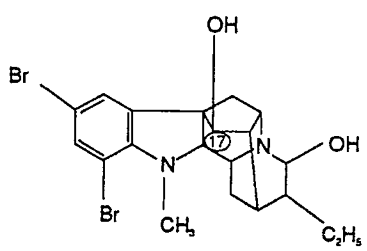

II

caractérisé en ce que l'on brome des composés de formule générale VI

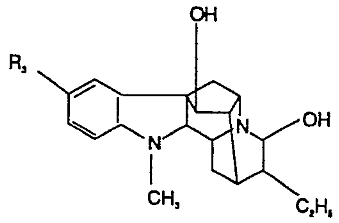

VI

dans laquelle $R_3$ est le brome ou l'hydrogène.